# FASCICULE DE BREVET EUROPEEN

(11) **EP 2 313 384 B1**
(45) Date de publication et mention de la délivrance du brevet: **18.09.2013**
(21) Numéro de dépôt: 09766076.5
(22) Date de dépôt: 16.06.2009
(51) Int. Cl.: C07D 295/073, A61K 31/495, A61P 19/02, A61P 29/00, A61P 37/00

(54) **PHENYL-ALKYL PIPERAZINES AYANT UNE ACTIVITE MODULATRICE DU TNF**
PHENYLALKYLPIPERAZINE MIT TNF-MODULIERENDER WIRKUNG
PHENYL-ALKYL PIPERAZINES HAVING A MODULATING ACTIVITY OF TNF

(30) Priorité: 16.06.2008 FR 0803337; 23.12.2008 FR 0807361
(43) Date de publication de la demande: 27.04.2011
(73) Titulaire: SANOFI, 75008 Paris (FR)
(72) Inventeur: BARONI, Marco, F-75013 Paris (FR); RITZELER, Olaf, 65926 Frankfurt (DE); ZANCHET, Marco, F-75013 Paris (FR)
(74) Mandataire: Veinante, Aude
(86) Numéro de dépôt international: PCT/FR2009/051138
(87) Numéro de publication internationale: WO 2009/153514

(56) Documents cités:
- WO-A-2004/060308
- JP-A- 2001 072 660
- HANANO T ET AL: "Novel phenylpiperazine derivatives as dual cytokine regulators with TNF-alpha suppressing and IL-10 augmenting activity" BIOORGANIC & MEDICINAL CHEMISTRY LETTERS, PERGAMON, ELSEVIER SCIENCE, GB, vol. 10, no. 9, 1 mai 2000 (2000-05-01), pages 875-879, XP004199033 ISSN: 0960-894X
- HANANO T ET AL: "Novel DMARDs on the basis of a new concept of dual cytokine regulation, TNF-alpha suppression and IL-10 augmentation" BIOORGANIC & MEDICINAL CHEMISTRY LETTERS, PERGAMON, ELSEVIER SCIENCE, GB, vol. 10, no. 9, 1 mai 2000 (2000-05-01), pages 881-884, XP004199034 ISSN: 0960-894X

## Description

La présente invention concerne de nouvelles phényl-alkyl-pipérazines ayant une activité modulatrice du TNF, les compositions pharmaceutiques les contenant et un procédé pour leur préparation.

Le TNF-alpha est une cytokine qui a été identifiée en tant que médiateur de l'immunité, de l'inflammation, de la prolifération cellulaire, de la fibrose, etc. Ce médiateur est copieusement présent dans le tissu synovial enflammé et exerce un rôle important dans la pathogenèse de l'auto-immunité (Annu. Rep. Med. Chem., 1997, 32:241-250).

Il a été maintenant trouvé que des phényl-alkyl-pipérazines possèdent une puissante activité vis-à-vis de la modulation du TNF-alpha, plus particulièrement une activité inhibitrice.

WO 2004 060308 et JP 2001 072660 décrivent des phenyl-alkyl-piperazines ayant une activité modulatrice du TNF-α qui diffèrent des composés de formule (I) présentement revendiqués en ce que le groupement R1 n'appartient pas à la liste revendiquée et le groupement CH₂CH₂R₃ est absent.

Des phenyl-alkyl-piperazines ayant une activité modulatrice du TNF-α sont décritent dans Bioorg. Med. Chem. Lett., 2000, 10(9), 875-9 et Bioorg. Med. Chem. Lett., 2000, 10(9), 881-4. Cependant elles portent un groupement -CH₂NHAc ou -NHAc en place du groupement -CH₂CH₂R₃ porté par celles revendiquées ici et aucune alternative n'est envisagée.

Ainsi, la présente invention concerne, selon un de ses aspects, des phényl-alkyl-pipérazines de formule (I) : 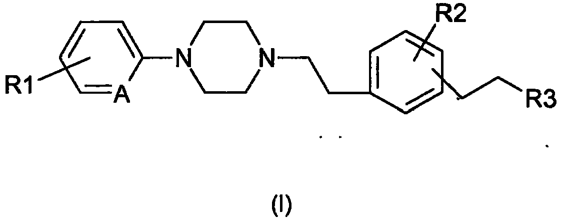 dans laquelle :
- R1 et R2 représentent, indépendamment l'un de l'autre, un atome d'hydrogène, un atome d'halogène, un groupe (C1-C5)alkyle, un groupe (C1-C5)haloalkyle, un groupe (C1-C2)perfluoroalkyle, un groupe (C1-C5)alcoxy ou un groupe (C1-C2)perfluoroalcoxy;
- R3 représente un groupe (C1-C5)alkyle ;
- A représente =CH- ou =N-.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels peuvent être préparés avec des acides pharmaceutiquement acceptables, mais les sels d'autres acides utiles, par exemple, pour la purification ou l'isolement des composés de formule (I) font également partie de l'invention.

Les composés de formule (I) peuvent exister comme dérivés N-oxydes. Effectivement, les composés de formule (I) peuvent notamment porter un ou deux groupes N-oxyde sur la pipérazine. Bien qu'en principe les deux azotes ci-dessus puissent tous être oxydés, les composés portant un seul N-oxyde, sont préférés.

Selon un autre objet de l'invention, on peut citer les composés de formule (I) dans lesquels:
- R1 représente un groupe (C1-C5)haloalkyle plus particulièrement un groupe (C1-C5)fluoroalkyle, un groupe (C1-C2)perfluoroalkyle; et/ou
- R2 représente un atome d'hydrogène ou un groupe (C1-C5)alkyle ; à l'état de base ou de sel d'addition à un acide.

Selon un autre objet de l'invention, la pipérazine est liée par le groupe éthyle en position 3 sur le groupe phényle: 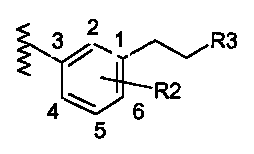 et/ou
- R1 représente un groupe (C1-C2)perfluoroalkyle; et/ou
- R2 représente un atome d'hydrogène ou un groupe (C1-C3)alkyle.

Selon un autre objet de l'invention, on peut citer les composés de formule (I) suivants:
1-(3-trifluorométhyl-phényl)-4-[2-(4-Méthyl-3-pentyl-phényl)-éthyl]-pipérazine ; à l'état de base ou de sel d'addition à un acide.

Selon un autre objet de l'invention, on peut citer un composé de formule (I) choisi parmi:
- composé n°1 :l'hémipamoate de 1-(3-trifluorométhyl-phényl)-4-[2-(4-Méthyl-3-pentyl-phényl)-éthyl]-pipérazine ;
- composé n°1 bis:l'hémipamoate de 1-(3-trifluorométhyl-phényl)-4-[2-(4-Méthyl-3-pentyl-phényl)-éthyl]-pipérazine en mélange avec 0, 5 mole d'acide pamoïque libre ;
- composé n°2 : le chlorhydrate de 1-(3-trifluorométhyl-phényl)-4-[2-(4-Méthoxy-3-pentyl-phényl)-éthyl]-pipérazine ;
- composé n°3 : le chlorhydrate de 1-(3-trifluorométhyl-phényl)-4-[2-(4-Méthyl-3-propyl-phényl)-éthyl]-pipérazine ;
- composé n°4 : le chlorhydrate de 1-(3-fluoro-phényl)-4-[2-(4-Méthyl-3-pentyl-phényl)-éthyl]-pipérazine ;
- composé n°5 : le chlorhydrate de 1-(3-trifluorométhyl-phényl)-4-[2-(4-Méthyl-3-heptyl-phényl)-éthyl]-pipérazine ;
- composé n°6 : le chlorhydrate de 1-(6-trifluorométhyl-pirid-2-yl)-4-[2-(4-Méthyl-3-pentyl-phényl)-éthyl]-pipérazine ;
- composé n°7 : le chlorhydrate de 1-(3-fluoro-phényl)-4-[2-(4-Méthoxy-3-pentyl-phényl)-éthyl]-pipérazine ;
- composé n°8 : le chlorhydrate de 1-(3-difluorométhyl-phényl)-4-[2-(4-Méthyl-3-pentyl-phényl)-éthyl]-piperazine ;
- composé n°9 : le chlorhydrate de 1-(phényl)-4-[2-(4-Méthyl-3-pentyl-phényl)-éthyl]-pipérazine ;
- composé n°10 : le chlorhydrate de 1-(3-méthoxy-phényl)-4-[2-(4-Méthyl-3-pentyl-phényl)-éthyl]-pipérazine ;
- composé n°11 : le chlorhydrate de 1-(3-trifluorométhyl-phényl)-4-[2-(4-Méthyl-3-(2-méthylbutyl)-phényl)-éthyl]-pipérazine ;
- composé n°12 : le chlorhydrate de 1-(3-difluorométhyl-phényl)-4-[2-(4-Méthoxy-3-pentyl-phényl)-éthyl]-pipérazine ;
- composé n°13 : le chlorhydrate de 1-(3-trifluorométhyl-phényl)-4-[2-(4-Méthoxy-3-heptyl-phényl)-éthyl]-pipérazine ;
- composé n°14 : le chlorhydrate de 1-(3-trifluorométhyl-phényl)-4-[2-(4-Méthyl-3-hexyl-phényl)-éthyl]-pipérazine ;
- composé n°15 : le chlorhydrate de 1-(3-trifluorométhyl-phényl)-4-[2-(4-Méthoxy-3-propyl-phényl)-éthyl]-pipérazine ;
- composé n°16 : le chlorhydrate de 1-(phényl)-4-[2-(4-Méthoxy-3-pentyl-phényl)-éthyl]-pipérazine ;
- composé n°17 : le chlorhydrate de 1- (4-chloro-phényl)-4-[2-(4-Méthyl-3-pentyl-phényl)-éthyl]-pipérazine ;
- composé n°18 : le chlorhydrate de 1-(3-trifluorométhyl-phényl)-4-[2-(4-Méthyl-3-pentyl-phényl)-éthyl]-pipérazine ;
- composé n°19 : le benzène sulphonate de 1-(3-trifluorométhyl-phényl)-4-[2-(4-Méthyl-3-pentyl-phényl)-éthyl]-pipérazine ;
- composé n°20 : le 2-naphtalène sulphonate de 1-(3-trifluorométhyl-phényl)-4-[2-(4-Méthyl-3-pentyl-phényl)-éthyl]-pipérazine ;
- composé n°21 : le p-Tolyl-sulphonate de 1-(3-trifluorométhyl-phényl)-4-[2-(4-Méthyl-3-pentyl-phényl)-éthyl]-pipérazine ;
- composé n°22 :l'hémi-2,5-naphtalène-disulphonate de 1-(3-trifluorométhyl-phényl)-4-[2-(4-Méthyl-3-pentyl-phényl)-éthyl]-pipérazine ;
- composé n°23 : le dichlorydrate de 1-(4-Fluoro-phényl)-4-[2-(4-méthyl-3-pentyl-phényl)-éthyl]-pipérazine ;
- composé n°24: la base de 1-(4-Méthoxy-phényl)-4-[2-(4-méthyl-3-pentyl-phényl)-éthyl]-pipérazine ;
- composé n°25 : le dichlorydrate de 1-(5-Bromo-pyridin-2-yl)-4-[2-(4-méthyl-3-pentyl-phényl)-éthyl]-pipérazine ;
- composé n°26 : le dichlorydrate de 1-[2-(4-Méthyl-3-pentyl-phényl)-éthyl]-4-(5-trifluorométhyl-pyridin-2-yl)-pipérazine;
- composé n°27: le chlorydrate de 1-(4-tert-Butyl-phényl)-4-[2-(4-méthyl-3-pentyl-phényl)-éthyl]-pipérazine ;
- composé n°28: le dichlorydrate de 1-(4-Ethoxy-phényl)-4-[2-(4-méthyl-3-pentyl-phényl)-éthyl]-pipérazine ;
- composé n°29 : le dichlorydrate de 1-[2-(4-Méthyl-3-pentyl-phényl)-éthyl]-4-m-tolyl-pipérazine ;
- composé n°30: le dichlorydrate de 1-[2-(4-Méthyl-3-pentyl-phényl)-éthyl]-4-pyridin-2-yl-pipérazine ;
- composé n°31 : le chlorydrate de 1-(6-Bromo-pyridin-2-yl)-4-[2-(4-méthyl-3-pentyl-phényl)-éthyl]-pipérazine ;
- composé n°32 : le chlorydrate de 1-(2-Chloro-phényl)-4-[2-(4-méthyl-3-pentyl-phényl)-éthyl]-pipérazine ;
- composé n°33 : le chlorydrate de 1-(2-méthyl-phényl)-4-[2-(4-méthyl-3-pentyl-phényl)-éthyl]-pipérazine ;
- composé n°34 : le chlorydrate de 1-(3-trifluorométhoxyl-phényl)-4-[2-(4-méthyl-3-pentyl-phényl)-éthyl]-pipérazine ;
- composé n°35: le chlorydrate de 1-(5-Chloro-pyridin-2-yl)-4-[2-(4-méthyl-3-pentyl-phényl)-éthyl]-pipérazine ;
- composé n°36: l'oxalate de 1-(3-trifluorométhyl-phényl)-4-[2-(4-Méthyl-3-pentyl-phényl)-éthyl]-pipérazine ;
- composé n°37 : le fumarate de 1-(3-trifluorométhyl-phényl)-4-[2-(4-Méthyl-3-pentyl-phényl)-éthyl]-pipérazine ;
- composé n°38 : le succinate de 1-(3-trifluorométhyl-phényl)-4-[2-(4-Méthyl-3-pentyl-phényl)-éthyl]-pipérazine ;
- composé n°39 : le dihippurate de 1-(3-trifluorométhyl-phényl)-4-[2-(4-Méthyl-3-pentyl-phényl)-éthyl]-pipérazine.

Dans le cadre de la présente invention, on entend par :
- un atome d'halogène : un fluor, un chlore, un brome ou un iode ;
- un groupe alkyle : un groupe aliphatique saturé linéaire ou ramifié. Par exemple, un groupe (C1-C5)alkyle comprend de 1 à 5 atomes de carbone. A titre d'exemples, on peut citer plus particulièrement les groupes méthyle, éthyle, propyle, isopropyle, butyle, isobutyle, tertbutyle , pentyle , isopentyle, ...
- un groupe haloalkyle : un groupe alkyle tel que défini ci-avant dont un ou plusieurs atomes d'hydrogène ont été substitués par un atome d'halogène, par exemple un groupe fluoroalkyle peut comporter un ou plusieurs atomes de fluor;
- un groupe perfluoroalkyle : un groupe alkyle tel que défini ci-avant dont tous les atomes d'hydrogène ont été substitués par un atome de fluor ;
- un groupe alcoxy : un groupe -O-alkyle où le groupe alkyle est tel que précédemment défini ;
- un groupe perfluoroalcoxy, un groupe alcoxy où tous les atomes d'hydrogène ont été substitués par un atome de fluor.

On entend par "groupe partant", dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un méthanesulfonate, benzènesulfonate, p-toluènesulfonate, triflate, acétate, etc. Des exemples de groupes partants ainsi que des références pour leur préparation sont donnés dans Advances in Organic Chemistry, J. March, 3rd Edition, Wiley Interscience, p. 310-316.

Les composés de formules (I) peuvent être préparés selon le schéma 1 : 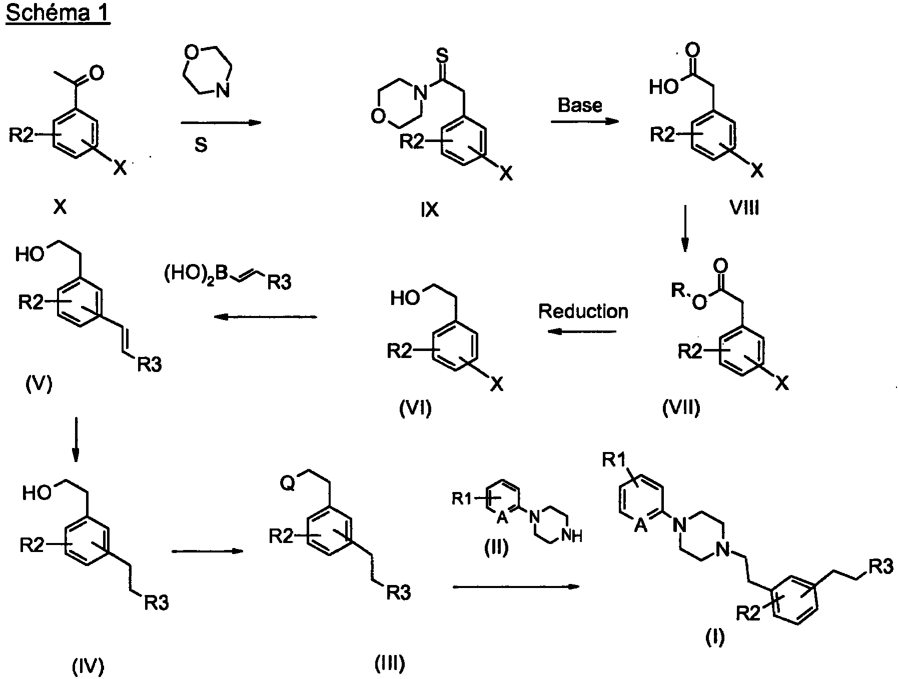 Selon ce schéma, les composés de formule (I) peuvent être synthétisés par condensation d'un composé de formule (II) 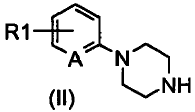 dans laquelle R1 et A sont définis comme précédemment, avec un composé de formule (III) 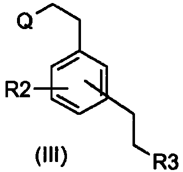 dans laquelle R2 et R3 sont tels que définis ci-dessus et Q est un groupe partant.

Comme groupe partant Q, on peut utiliser par exemple un atome d'halogène ou tout groupe apte à la condensation avec une amine. La réaction de condensation est réalisée de manière conventionnelle par mélange des composés de départ (II) et (III) dans un solvant organique tel qu'un alcool, par exemple le méthanol ou le butanol, éventuellement en présence d'une base telle qu'un carbonate alcalin, la 4-diméthylamino-pyridine ou la triéthylamine, à une température comprise entre la température ambiante et celle de reflux du solvant choisi. Par température ambiante, on entend une température comprise entre 5 et 25°C.

Le composé de formule (III) est préparé par transformation de l'hydroxy du composé de formule (IV) en groupe partant Q selon des méthodes classiques connues de l'homme du métier. Par exemple, l'hydroxy peut être transformé en halogène tel qu'un brome en présence d'acide bromique, de tétrabromure de carbone ou de bromure de thionyle ; ou bien tel qu'un chlore en présence de chlorure de thionyle. On peut ainsi préparer comme groupe partant le groupe mésyloxy (CH₃-SO₂-O-) ou tosyloxy (tolyl-SO2-O) par réaction des produits de formule (IV) avec le chlorure de méthanesulphonyl ou de p-tolylsulphonyl.

Le composé (IV) est obtenu par réduction de la double liaison du composé de formule (V). La réduction peut s'effectuer selon des méthodes classiques connues de l'homme, du métier, par exemple en présence d'hydrogène sur catalyse palladium/charbon dans un solvant organique tel que l'éthanol à une température comprise entre 20 et 40°C.

Le composé de formule (V) peut être préparé par réaction de Suzuki (J. Org. Chem., 55 6184 -1990). Selon cette méthode, on fait réagir un composé de formule (VI) dans lequel X représente un halogène, plus particulièrement un brome ou un iode ; ou un trifluorométhylsulphonate avec un acide vinyl boronique ou un vinyl boronate en présence d'un catalyseur tel que l'acétate de palladium ou le palladium de tétrakis(triphénylphosphine) dans un solvant tel que le tétrahydrofurane, le dioxane ou le diméthoxiéthane.

Le composé de formule (VI) peut être obtenu à partir du composé de formule (X) selon les étapes décrites dans le schéma 1 selon des conditions bien connues de l'homme du métier et analogues ou telles que détaillées dans les exemples.

Les composés de formule (II) sont commerciaux ou bien ils peuvent être préparés par des méthodes bien connues à l'homme du métier.

Dans le schéma, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'homme du métier.

Le procédé selon l'invention peut également comprendre l'étape d'isolement et/ou purification du composé de formule (I) ainsi obtenu et/ou sa transformation éventuelle en un de ses sels et/ou son N-oxyde.

Le composé voulu est isolé selon les techniques conventionnelles sous forme de base libre ou d'un de ses sels. La base libre peut être transformée dans un de ses sels par simple salification dans un solvant organique tel qu'un alcool, de préférence l'éthanol ou l'isopropanol, un éther comme le 1,2-diméthoxyéthane, l'acétate d'éthyle, l'acétone ou un hydrocarbure comme l'hexane.

Les composés de formule (I) portant un groupe N-oxyde sur les atomes d'azote peuvent être préparés par oxydation des composés de formule (I) correspondants. Dans ce cas, le composé de formule (I) tel qu'obtenu par les synthèses ci-dessus est soumis à une réaction d'oxydation selon les méthodes conventionnelles, par exemple à une réaction avec de l'acide m-chloro-perbenzoïque dans un solvant convenable et isolé selon les techniques usuelles bien connues de l'homme du métier.

Les exemples suivants décrivent la préparation d'un composé conforme à l'invention. Ces exemples ne sont pas limitatifs et ne font qu'illustrer la présente invention. Le numéro du composé exemplifié renvoie à celui donné dans le tableau ci-après, qui illustre la structure chimique et les propriétés physiques du composé selon l'invention.

Les mesures physico-chimiques ont été effectuées de la façon suivante :
Les points de fusion ont été mesurés avec un appareil Buchi B540.

Les spectres de résonance magnétique nucléaire du proton (RMN ¹H) ont été enregistrés à 500 MHz sur un appareil Bruker équipé avec une console Avance. Les déplacements chimiques sont rapportés en ppm par rapport à la fréquence TMS.

Tous les spectres ont été enregistrés à la température de 40°C.

Les abréviations utilisées pour caractériser les signaux sont les suivantes: s = singulet, bs =singulet large, m = multiplet, bm= multiplet large, d = doublet, t = triplet, q = quadruplet.
• = non intégrable à cause de l'interférence avec un pic large du à l'eau.

Pour la technique LC/MS :
Système ThermoElectron LCQ Deca XP Max équipé avec un détecteur à spectrométrie de masse à trappe d'ions ainsi qu'un détecteur à barre de diodes.

Les systèmes chromatographiques utilisés sont les suivants:
Méthode A)
   - colonne Kromasil C18 (2,1 x 50 mm) 3,5µm
   - Eluant A = H₂O acétate d'ammonium pH 6,5 5mM
   - Eluant B = CH₃CN.
   - Gradient de 98% de A à 95% de B en 12 minutes, puis élution à 95% de B pendant 3 minutes
   - Débit 0,5ml/minute
   - Injection de 2µL de solution à 0,1mg/ml dans un mélange CH₃CN : H₂O = 9 :1
Méthode B)
   - colonne Kromasil C18 (2,1 x 50 mm) 3,5µm
   - Eluant A = H₂O + 0,01% TFA
   - Eluant B = CH₃CN.
   - Gradient de 98% de A à 95% de B en 15 minutes, puis élution à 95% de B pendant 5 minutes
   - Débit 0,5ml/minute
   - Injection de 2µL de solution à 0,1mg/ml dans un mélange CH₃CN : H₂O = 9 :1
Méthode C)
   - colonne Varian Sunfire C18 (2,0 x 100 mm) 3,5µm
   - Eluant A = H₂O + 0,01 % TFA
   - Eluant B = CH₃CN.
   - Gradient de 98% de A à 95% de B en 15 minutes, puis élution à 95% de B pendant 5 minutes
   - Débit 0,5ml/minute
   - Injection de 2µL de solution à 0,1mg/ml dans un mélange CH₃CN : H₂O = 9 :1
Méthode D)
   - colonne Waters Atlantis DB C18 (2,0 x 50 mm) 3,0µm
   - Eluant A = H₂O + 0,01% TFA
   - Eluant B = CH₃CN.
   - Gradient de 98% de A à 95% de B en 15 minutes, puis élution à 95% de B pendant 5 minutes
   - Débit 0,5ml/minute
   - Injection de 2µL de solution à 0,1mg/ml dans un mélange CH₃CN : H₂O = 9 :1
Méthode E)
   - colonne XTerra C18 (2,1 x 50 mm) 3,5µm n°186000400
   - Eluant A = H₂O + 0,01% TFA
   - Eluant B = CH₃CN.
   - Gradient de 98% de A à 95% de B en 15 minutes, puis élution à 98% de B pendant 5 minutes.
   - Débit 0,5ml/minute
   - Injection de 2µL de solution à 0,1mg/ml dans un mélange CH₃CN : H₂O = 9 :1
Méthode F)
   - colonne Ascentis C18 2x50mm 3µm
   - Eluant A = H₂O + 0,01% TFA
   - Eluant B = CH₃CN
   - Gradient de 98% de A à 95% de B en 10 minutes, puis élution par 95% de B pendant 5 minutes.
   - Débit 0,5 ml/minute; température 40°C
   - Injection de 2 µL de solution à 0,1 mg/ml dans un mélange CH₃CN : H₂O = 9 :1
Méthode G)
   - colonne Ascentis C18 2x50mm 3µm
   - Eluant A = H₂O + 0,05% TFA
   - Eluant B = CH₃CN + 0.035 TFA
   - Gradient de 98% de A à 95% de B en 12 minutes, puis élution par 95% de B pendant 3 minutes.
   - Débit 0,7 ml/minute; température 40°C
   - Injection de 2 µL de solution à 0,1 mg/ml dans un mélange CH₃CN : H₂O = 9 :1

Les produits sont détectés en UV à 220 nm.

Pour la partie spectrométrie de masse :
- Mode d'ionisation: electrospray positif (ESI+ polarité+)
- Balayage de 100 à 1200 uma

Le gel de silice pour la chromatographie sur colonne flash est commercialisé par Biotage.

### PREPARATION 1 4-(2-Bromo-éthyl)-1-méthyl-2-pentyl-benzène

### 1a) Acide (3-bromo-4-méthyl-phényl)-acétique

On mélange 13 g (0,061 mole) de 3-bromo-4-méthyl-acétophénone, 2,1 g (0,065 mole) de soufre, 14 ml de morpholine et une quantité catalytique d'acide p-toluènsulfonique monohydraté. On chauffe sous courant d'azote à 130°C. Après 7 heures, on refroidit, on ajoute 35 ml d'éthanol absolu et on agite à température ambiante pendant une nuit. On dissout 13,9 g du thioamide ainsi formé dans une solution de 110 ml d'éthanol, 70 ml d'eau et 6 g de NaOH et on chauffe au reflux pendant 4 heures. On évapore les solvants et on acidifie ensuite avec une solution diluée d'acide chlorhydrique. On obtient la précipitation d'un solide blanc. On filtre et obtient 9,16 g du composé du titre.

### 1 b) 2-(3-Bromo-4-méthyl-phényl)-éthanol

On dissout le composé de la préparation 1a) dans 170 ml d'éthanol. On fait barboter de l'acide chlorhydrique gazeux pendant 30 minutes. On chauffe à reflux pendant 3 heures. On évapore l'éthanol et on reprend avec de l'éther diéthylique. On lave avec une solution saturée de bicarbonate de sodium et on évapore sous vide. On obtient 7,1g d'ester qui sont dissouts dans 70 ml de THF. On ajoute goutte à goutte une solution de 7,6 ml de diméthylsulfure de borane dans 110 ml de THF, sous azote, et on chauffe à reflux pendant 3 heures. On refroidit à 0 °C et on ajoute avec précaution 120 ml de méthanol. On chauffe à reflux pendant 30 minutes. On évapore sous vide. Le résidu est dissout dans de l'acétate d'éthyle. On lave avec une solution diluée d'ammoniaque, on sèche et on évapore sous vide.

On obtient 5,4 g sous forme d'une huile correspondant au composé du titre.

### 1c) 2-[4-Méthyl-3-(pent-1-enyl)-phényl]-éthanol

On mélange 2,0 g (0,0093 mole) de produit de l'étape précédente, 1,12 g (0,01 mole) d'acide pentenyl-boronique, 2,1 g (0,037 mole) de KOH, 1,5 g (0,0046 mole) de bromure de tetrabutylammonium et 50 mg de Pd-tétrakis(triphénylphosphine) dans 50 ml de THF. On chauffe à reflux sous courant d'azote pendant 4 heures. On verse le mélange dans de l'eau, on extrait à l'éther diéthylique, on sèche la phase organique et on évapore le solvant. On purifie le résidu par chromatographie sur colonne de gel de silice en éluant par un mélange hexane/acétate d'éthyle 9/1. On obtient 740 mg de produit du titre sous forme d'huile.

### 1d) 2-(4-Méthyl-3-pentyl-phényl)-éthanol

Le produit de l'étape précédente, 0,74 g (0,0036 mole), est solubilisé dans 46 ml d'éthanol, on ajoute 0,12 g de Pd/C à 10% et on laisse réagir sous flux d'hydrogène pendant 5 heures à la température de 40°C. On filtre et on évapore sous vide. On obtient 0,65 g du composé du titre sous forme d'huile.

### 1e) 4-(2-Bromo-éthyl)-1-méthyl-2-pentyl)-benzène

Le produit de l'étape précédente, 0,65 g (0,0032 mole), est chargé dans un ballon avec 8 ml d'une solution aqueuse d'acide bromhydrique au 48%. On chauffe à 130°C pendant 6 heures. On refroidit et on verse dans une solution saturé de bicarbonate de sodium. On extrait avec de l'acétate d'éthyle, on sèche et on évapore sous vide. On obtient 0,65 g du composé du titre sous forme d'huile.

### PREPARATION 2 4-(2-Méthanesulphonyloxy-éthyl)-1-méthoxy-2-pentyl-benzène

### 2a) Acide (3-bromo-4-méthoxy-phényl)-acétique

En opérant comme décrit pour la préparation 1a) mais en utilisant le 3-bromo-4-méthoxy-acétophénone au lieu du 3-bromo-4-méthyl -acétophénone, on obtient le composé du titre.

### 2b) 2-(3-Bromo-4-méthoxy-phényl)-éthanol

En opérant comme décrit pour la préparation 1b) mais en utilisant le composé 2a) de l'étape précédente au lieu du composé 1a), on obtient le composé du titre.

### 2c) 2-[4-Méthoxyl-3-(pent-1-enyl)-phényl]-éthanol

En opérant comme décrit pour la préparation 1c) mais en utilisant le composé 2b) de l'étape précédente au lieu du composé 1b), on obtient le composé du titre.

### 2d) 2-(4-Méthoxy-3-pentyl-phényl)-éthanol

En opérant comme décrit pour la préparation 1d) mais en utilisant le composé 2c) de l'étape précédente au lieu du composé 1c), on obtient le composé du titre.

### 2e) 4-(2-Méthanesulphonyloxy-éthyl)-1-méthoxy-2-pentyl-benzène

On charge dans un ballon 1,2 g (0,0054 mole) du composé 2d) de l'étape précédente, 20 ml de dichlorométhane, 0,75 ml (0,0054 mole) de triéthylamine et, à 0-5°C, 0,42 ml (0,0054 mole) de méthanesulphonyl chlorure.

On agite pendant 2 heures à température ambiante. On lave avec de l'eau, on sèche et on évapore sous vide la phase organique. On obtient le produit du titre sous forme d'une huile jaune.

### Exemple 1 : composé n°1 :1-[2-(4-Méthyl-3-pentyl-phényl)-éthyl]-4-(3-trifluorométhyl-phényl)-pipérazine et son hémipamoate

On charge dans un ballon 1,98 g (0,00777 mole) du composé de la préparation 1, 1,5 g (0,0065 mole) de (3-trifluorométhylphényl)-1-pipérazine, 1,35 g (0,00975 mole) de carbonate de potassium et 40 ml de n-butanol. On chauffe à reflux pendant 6 heures. On évapore le n-butanol, on reprend avec de l'acétate d'éthyle, on lave avec de l'eau, on sèche la phase organique et on évapore le solvant. On purifie le résidu par chromatographie sur colonne de gel de silice en éluant par un mélange hexane/acétate d'éthyle 95/5.

On mélange la solution de 300 mg de la base libre obtenue en THF avec une solution de 180 mg d'acide pamoïque en THF/eau 8:2.

On concentre sous vide. On ajoute 6 ml d'éthanol et on obtient la formation d'un solide que l'on filtre. On cristallise en chauffant avec de l'éthanol et on obtient l'hémipamoate blanchâtre: 450 mg avec p.f = 156-157. Une cristallisation ultérieure par éthanol permet d'obtenir un p.f. de 157-158.
Point de fusion = 157-158°C ;
M+H⁺ (Méthode A) = RT 10,9 min m/z 419 (MH⁺)
**RMN ¹H** : δ (ppm, dmso-d6): 0,88 (m; 3H); 1,27-1,39 (m ; 4H); 1,45-1,59 (m; 2H); 2,23 (s; 3H); 2,50-2,58 (m; 2H**); 2,75-2,89 (m; 2H); 2,89-3,50 (m; 10H*); 4,74 (s; 1H); 6,97 (dd; Ja= 7,7 Hz; Jb= 1,4 Hz; 1H); 7,02 (d; J= 1,4 Hz; 1H); 7,07 (d; J= 7,7 Hz; 1H); 7,08-7,15 (m; 2H); 7,16-7,32 (m; 3H); 7,45 (dd→t; J= 8 Hz; 1H); 7,74 (d; J= 8 Hz; 1 H); 8,17 (d; J= 8 Hz; 1 H); 8,30 (s ; 1 H).

Selon l'une des variantes de la présente invention, on prépare le composé 1 bis, mélange d'hémipamoate de 1-[2-(4-Méthyl-3-pentyl-phényl)-éthyl]-4-(3-trifluorométhyl-phényl)-pipérazine et d'acide pamoïque libre (ratio molaire 1 :0, 5) selon la méthode suivante :
On mélange 600 mg de la base libre obtenue selon la méthode de l'exemple 1 en solution de THF avec 540 mg d'acide pamoïque en solution de THF/eau 8 :2.
On évapore sous vide puis on traite le résidu avec un mélange de THF et d'éther isopropylique. On chauffe et on filtre. On obtient 1 g de solide jaune.
Point de fusion = 158-159°C ;
M+H⁺ (Méthode B) = RT 10,1 min m/z 419 (MH⁺)

### Exemple 2: composé n°2 :1-[2-(4-Méthoxy-3-pentyl-phényl)-éthyl]-4-(3-trifluorométhyl-phényl)-pipérazine et son chlorhydrate

On charge dans un ballon 0, 336 g (0,00112 mole) de composé de la préparation 2, 20 ml d'isopropanol, 0,47 ml de triéthylamine (0,0036 mole), et 0,21 ml (0,00112 mole) de 4-(3-trifluorométhyl-phényl)-pipérazine. On chauffe à la température de reflux pendant une nuit. On évapore le solvant sous vide et on obtient une huile que l'on purifie par chromatographie en éluant avec un mélange hexane/éthyle acétate 8 :2. On obtient 300 mg de base libre avec laquelle on prépare le chlorhydrate en isopropanol à l'aide une solution d'isopropanol saturée avec HCl.

On filtre et on obtient 100 mg d'un solide blanc.

Point de fusion = 146-149° C
M+H⁺ (Méthode B)= RT 7.0 min m/z 435 (MH⁺)
**RMN ¹H**: δ (ppm, dmso-d6): 0,88 (m; 3H); 1,23-1,38 (m ; 4H); 1,53 (m; 2H); 2,51-2,57 (m; 2H**); 2,95-3,05 (m; 2H); 3,11-3,28 (m; 4H); 3,30-3,41 (m; 2H); 3,60-3,70 (m; 2H*); 3,77 (s; 3H); 3,93-4,04 (m; 2H); 6,92 (d; J= 8 Hz; 1 H); 7,05 (d; J= 2 Hz; 1H); 7,07 (dd; Ja= 8 Hz; Jb= 2 Hz; 1H); 7,17 (bd; J= 7 Hz ;1H); 7,29 (bs; 1 H); 7,31 (bd ; J= 8 Hz; 1 H); 7,49 (dd→t; J= 8 Hz; 1 H); 10,59 (bs; 1 H).

### Exemple 3: composé n°38:1-[2-(4-Méthyl-3-pentyl-phényl)-éthyl]-4-(3-trifluorométhyl-phényl)-pipérazine et son oxalate

On charge dans un ballon 0,24 g (0,89 mmole) du composé de la préparation 1, 0,24 g (0,1 mmole) de (3-trifluorométhylphényl)-1-pipérazine, 0,22 g (1,6 mmole) de carbonate de potassium et 10 ml de n-butanol. On chauffe à reflux pendant 6 heures. On évapore le n-butanol, on reprend avec de l'acétate d'éthyle, on lave avec de l'eau, on sèche la phase organique et on évapore le solvant. On purifie le résidu par chromatographie sur colonne de gel de silice en éluant par un mélange hexane/acétate d'éthyle 95/5. On obtient ainsi 130 mg de produit du titre sous forme d'huile. On dissout le produit dans 2 ml d'isopropanol. On ajoute une solution d'acide oxalique dans l'isopropanol et on obtient la précipitation de l'oxalate qui est isolé sous forme d'un solide blanc par filtration (0,12g).
Point de fusion = 193-194°C ;
M+H⁺ = RT 6,7 min m/z 419 (MH⁺)
RMN ¹H δ (ppm, dmso-d6): 0,89 (m; 3H); 1,28-1,40 (m; 4H); 1,46-1,59 (m; 2H); 2,23 (s; 3H); 2,50-2,57 (m;**); 2,78-2,89 (m;2H); 2,91-3,08 (m;*); 3,39 (bs; 4H); 6,97 (dd; Ja= 7,7Hz; Jb= 1,6Hz; 1 H); 7,02 (bs; 1 H); 7,07 (d; J= 7,7Hz; 1 H); 7,12 (d;J= 7,4Hz; 1 H); 7,22 (bs; 1 H); 7,27 (bd; J= 8,4Hz; 1 H); 7,45 (m; 1 H).

### TABLEAU

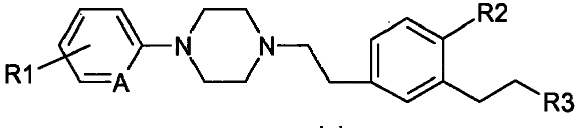

| **N°** | 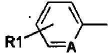 | **R2** | **R3** | **Sel** | **PF°C** | **M+ H+** |
|---|---|---|---|---|---|---|
| 1 | 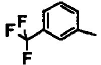 | 4-CH₃ | 3-n-C₃H₇ | 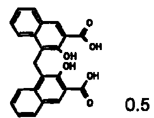 | 157-158°C | MH+ 419 RT 10,9 Méthode A |
| 1 bis | 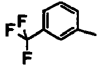 | 4-CH₃ | 3-n-C₃H₇ | 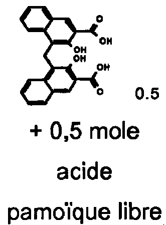 | 158-159°C | MH+ 419 RT 10,1 Méthode B |
| 2 | 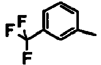 | 4-OCH₃ | 3-n-C₃H₇ | HCl | 146-149°C | MH+ 435 RT 7,0 Méthode B |
| 3 | 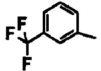 | 4-CH₃ | 3-CH₃ | HCl | 198-200°C | MH+ 391 RT. 6,6 Méthode B |
| 4 | 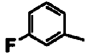 | 4-CH₃ | 3-n-C₃H₇ | HCl | 215-217°C | MH+ 369 RT. 7,4 Méthode B |
| 5 | 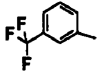 | 4-CH₃ | 3-n-C₅H₁₁ | HCl | 190-192°C | MH+ 447 RT 7,2 Méthode C |
| 6 | 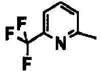 | 4-CH₃ | 3-n-C₃H₇ | HCl | 161-163°C | MH+ 420 RT. 7,6 Méthode B |
| 7 | 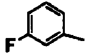 | 4-OCH₃ | 3-n-C₃H₇ | HCl | 174-177°C | MH+ 385 RT 6,7 Méthode B |
| 8 | 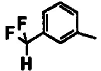 | 4-CH₃ | 3-n-C₃H₇ | HCl | 182-185°C | MH+ 401 RT. 10,2 Méthode D |
| 9 | 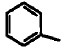 | 4-CH₃ | 3-n-C₃H₇ | HCl | 200-202°C | MH+ 351 RT. 6,9 Méthode B |
| 10 | 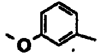 | 4-CH₃ | 3-n-C₃H₇ | HCl | 199-201°C | MH+ 381 RT 7,1 Méthode B |
| 11 | 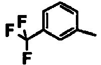 | 4-CH₃ | 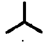 | HCl | 149°C | MH+ 433 RT 10,2 Méthode B |
| 12 | 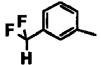 | 4-OCH₃ | 3-n-C₃H₇ | HCl | 125-128°C | MH+ 417 RT. 6,9 Méthode D |
| 13 | 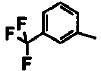 | 4-OCH₃ | 3-n-C₅H₁₁ | HCl | 214°C | MH+ 463 RT. 10,3 Méthode A |
| 14 | 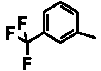 | 4-CH₃ | 3-n-C₄H₉ | HCl | 187°C | MH+ 433 RT. 7,2 Méthode B |
| 15 | 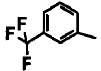 | 4-OCH₃ | 3-CH₃ | HCl | 184-185°C | MH+ 407 RT. 6,1 Méthode C |
| 16 | 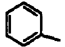 | 4-OCH₃ | 3-n-C₃H₇ | HCl | 171-173°C | MH+ 367 RT 6,5 Méthode B |
| 17 | 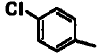 | 4-CH₃ | 3-n-C₃H₇ | HCl | 203°C | MH+ 385 RT 10,9 Méthode A |
| 18 | 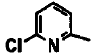 | 4-CH₃ | 3-n-C₃H₇ | HCl | 180-181°C | MH+ 386 RT 10,6 Méthode B |
| 19 | 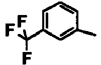 | 4-CH3 | 3-n-C5H11 | 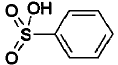 | 165°C | MH+ 419 RT 7,2 Méthode B |
| 20 | 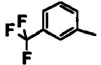 | 4-CH3 | 3-n-C5H11 | 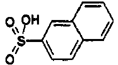 | 160-164°C | MH+ 419 RT 7,2 Méthode B |
| 21 | 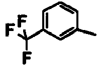 | 4-CH3 | 3-n-C5H11 | 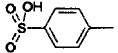 | 163-165°C | MH+ 419 RT 7,1 Méthode B |
| 22 | 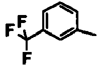 | 4-CH3 | 3-n-C5H11 | 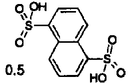 | 283-285°C | MH+ 419 RT 7,5 Méthode B |
| 23 | 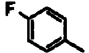 | 4-CH3 | 3-n-C5H11 | 2*HCl | >201 | MH+ 369 RT 7,3 Méthode G |
| 24 | 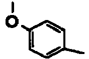 | 4-CH3 | 3-n-C5H11 | - | 68-70 | MH+ 381 RT 6,3 Méthode F |
| 25 | 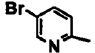 | 4-CH3 | 3-n-C5H11 | 2*HCl | 190-192 | MH+ 430 RT 6,5 Méthode F |
| 26 | 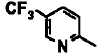 | 4-CH3 | 3-n-C5H11 | 2*HCl | >212 | MH+ 420 RT 7,6 Méthode G |
| 27 | 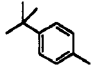 | 4-CH3 | 3-n-C5H11 | HCl | 193-195 | MH+ 407 RT 8,3 Méthode G |
| 28 | 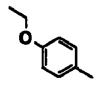 | 4-CH3 | 3-n-C5H11 | 2*HCl | 228-230 | MH+ 395 RT 7,38 Méthode G |
| 29 | 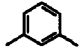 | 4-CH3 | 3-n-C5H11 | 2*HCl | 226-228 | MH+ 365 RT 7,1 Méthode G |
| 30 | 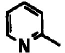 | 4-CH3 | 3-n-C5H11 | 2*HCl | 183-185 | MH+ 352 RT 5,5 Méthode E |
| 31 | 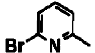 | 4-CH3 | 3-n-C5H11 | HCl | 185-187 | MH+ 430 RT 6,5 Méthode G |
| 32 | 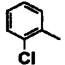 | 4-CH3 | 3-n-C5H11 | HCl | 200-202 | MH+ 385 RT 7,1 Méthode E |
| 33 | 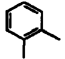 | 4-CH3 | 3-n-C5H11 | HCl | 220-222 | MH+ 365 RT 7,6 Méthode E |
| 34 | 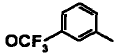 | 4-CH3 | 3-n-C5H11 | HCl | 197-200 | MH+ 435 RT 7,8 Méthode E |
| 35 | 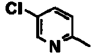 | 4-CH3 | 3-n-C5H11 | HCl | 199-201 | MH+ 386 RT 7,8 Méthode E |
| 36 | 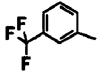 | 4-CH₃ | 3-n-C₃H₇ | Oxalate | 193-194 | MH+419 RT 6,7 Méthode E |
| 37 | 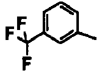 | 4-CH₃ | 3-n-C₃H₇ | fumarate | 140 | MH+419 RT 6,9 Méthode E |
| 38 | 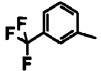 | 4-CH₃ | 3-n-C₃H₇ | Succinate | 95-96 | MH+419 RT 6,6 Méthode E |
| 39 | 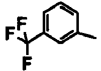 | 4-CH₃ | 3-n-C₃H₇ | di-hippurate | 91-92 | MH+419 RT 6,7 Méthode E |

Les composés de l'invention possèdent des propriétés intéressantes vis-à-vis de l'inhibition du TNF-α.

Ces propriétés ont été mises en évidence à l'aide d'un test visant à mesurer l'effet de molécules sur la synthèse du TNF-α induite chez la souris Balb/c par du lipopolysaccharide (LPS) d'Escherichia Coli (055 :B5, Sigma, St Louis, Mo).

Les produits à tester sont administrés par voie orale à des groupes de cinq souris Balb/c femelles (Charles River, France) âgées de 7 à 8 semaines. Une heure après, le LPS est administré par voie intraveineuse (10µg/souris). Le sang de chaque animal est prélevé 1,5 heure après l'administration du LPS. Les échantillons sont centrifugés, le plasma est récupéré et congelé à -80°C. Le TNF-α est mesuré à l'aide de kits commerciaux (R et D, Abingdon, UK).

Dans ce test, le composé 38 s'est montré très actif, en inhibant la synthèse du TNF-α même à doses très faibles, IC50= 0,1 mg/Kg

Ces propriétés ont également été mises en évidence à l'aide d'un test visant à mesurer l'effet de molécules sur la synthèse du TNF-α induite chez le rat Sprague-Dawlay par du lipopolysaccharide (LPS) d'Escherichia Coli (055 :B5, Sigma, St Louis, Mo).

Les produits à tester sont administrés par voie orale à des groupes de dix rats Sprague-Dawlay males pesant environ 200 grammes. Deux heures après, le LPS est administré par voie intraveineuse (0,1mg/Kg). Le sang de chaque animal est prélevé 1,5 heure après l'administration du LPS. Les échantillons sont centrifugés, le sérum est récupéré et congelé à -80°C. Le TNF-α est mesuré à l'aide de kits commerciaux (RPN 2744 Amersham, UK).

Dans ce test, les composés se sont montrés très actifs, en inhibant la synthèse du TNF-α même à doses très faibles, IC50= de 0,3 à 1mg/Kg, notamment une IC50=0,3mg/Kg pour le composé 1 et une IC50=1 mg/Kg pour les composés 4 et 8.

Les composés de l'invention sont testés dans un modèle d'inflammation articulaire.

Le composé (1ng/articulation en suspension dans 10µl d'une solution à 2% PVP(Polyvinylpyrolidone)/ 1% lutrol F68/ 0,9 % NaCl) selon l'invention est injecté une heure avant la première injection de zymosan® (voir ci-après) dans l'articulation d'un hamster.

Induction de l'inflammation articulaire du genou : Sous anesthésie légère par Isofluran, une suspension de zymosan ® à une dose de 100 µg dans 10µl de solution saline, est injecté dans l'articulation du genou de l'hamster mâle. Le zymosan® est un extrait de levure qui produit une forte inflammation dose-dépendante quand injecté en sous-cutané. Dans les conditions expérimentales décrites ici (injection dans l'articulation du genou), il induit une hyperalgésie avec une durée d'une semaine. Pour prolonger cette durée, 3 injections consécutives de zymosan® ont été appliquées. Le temps de latence du retrait de la patte, suite à l'exposition de la peau plantaire à un stimulus thermique défini, est mesuré en utilisant un appareil (Plantar Test Ugo Basile Biological Research Apparatus, Comerio, Italie) comprenant une mini caméra afin d'assurer le positionnement exact du rayon infrarouge en dessous de la patte arrière concernée.

### Mesure de l'hyperalgésie secondaire :

Le minuteur qui mesure la durée de la lumière infrarouge réfléchie par la patte arrière est démarré par l'investigateur et s'arrête automatiquement dès que l'animal secoue ou retire sa patte. La lumière infrarouge est arrêtée après 16 secondes par l'investigateur dans le cas où l'animal ne retire pas la patte afin d'éviter une brûlure. Le temps de latence du retrait de la patte en secondes est utilisé comme mesure de la douleur. La mesure a été réalisée 4 heures après la première injection du zymosan® et pendant les 3 semaines suivantes. Dans ce test, des composés représentatifs de l'invention et notamment le composé 1 montre une réduction efficace de la douleur sur une période de 3 semaines.

Les composés de l'invention sont également testés dans un modèle de douleur neuropathique : mesure de l'effet anti-allodynique dans la blessure du nerf épargnée (SNI).

Dans ce modèle in vivo de souris souffrant de douleur neuropathique chronique, l'effet sur l'allodynie tactile, une sensation douloureuse induite après un stimulus mécanique normalement non douloureux, est incité dans une patte arrière par lésion chirurgicale du grand nerf sciatique. Il est ensuite déterminé en mesurant le seuil de retrait de la patte (PWT), c'est-à-dire la force exercée (exprimé en grammes) à laquelle la souris a retiré la patte arrière.

L'allodynie tactile est déterminée sur les deux pattes arrières, c'est-à-dire homolatérale et controlatérale à la lésion déterminée à la suite de la chirurgie, avec l'essai automatique von Frey, dans lequel la peau plantaire des pattes arrières est exposée à un stimulus de pression d'intensité croissante jusqu'à 5 grammes, en utilisant un bâton d'aiguille de décharge. La force en grammes à laquelle l'animal a répondu par le retrait de la patte de derrière (le seuil de retrait de patte, PWT), est utilisée comme une mesure d'allodynie tactile.

Des groupes d'essai avec les composés selon l'invention et un groupe témoin d'animaux ont été utilisés, chacun étant constitué de quatre souris mâle C57B6. Dans tous les groupes, la ligne de base des valeurs de PWT (BL) a été déterminée avant la chirurgie. Sous anesthésie générale et afin d'induire la douleur neuropathique, les deux branches majeures du grand nerf sciatique sont ligaturées et sectionnées transversalement dans tous les groupes, avec le nerf saphène externe laissé intact (la blessure de nerf épargnée, SNI). Dans ce modèle de douleur neuropathique, l'allodynie tactile s'est complètement développée dans les deux jours suivant la section transversale de nerf dans la patte arrière homolatérale à la lésion et reste stable dans le groupe témoin au cours de la période d'observation de l'expérience.

7 jours après la chirurgie, immédiatement avant l'administration du composé, c'est-à-dire au temps 0 h, et dans tous les groupes, les valeurs PWT ont été déterminées pour la patte arrière homolatérale (blessée) et la patte arrière controlatérale (non-blessée). Les composés selon l'invention ou le véhicule ont été administrés aux animaux par injection dans la veine de queue (i.v). Dans les groupes d'essai, les souris ont reçu 0,1 ml/10 g d'une solution des composés d'essai dans le véhicule (Ethanol/Cremophor/Phosphate Buffered Salin 1:1:18) à une posologie de 1 mg/kg. Dans le groupe témoin, les souris ont de façon analogue reçu 0,1 ml/10g du véhicule. 1 h, 2 h, 4 h et 6 h après l'administration, les valeurs PWT ont été déterminées.

Pour l'analyse statistique, les valeurs de PWT mesurées ont été analysées par ANOVA à 1 voie et la Comparaison Multiple Bonferroni. À la dose évaluée (1mg/kg i.v.) une réduction de douleur statistiquement significative (par mesure de l'effet anti-allodynique) a été observée pour l'exemple 1 de 1 h à 6 h.

Grâce à cette activité, les composés de formule (I) et ses sels peuvent être utilisés dans le traitement des maladies liées à des troubles immunitaires et inflammatoires ou comme analgésiques pour le traitement de la douleur.

Notamment les composés de formule (I) peuvent être utilisés pour traiter l'athérosclérose, les maladies auto-immunes, les maladies qui entraînent la démyélinisation des neurones (telles que la sclérose en plaques), l'asthme, les douleurs ou inflammations articulaires plus particulièrement celles de l'épaule, du genou, des doigts,..., l'arthrite rhumatoïde et ses douleurs articulaires, l'ostéoarthrite et ses douleurs articulaires ainsi que d'autres douleurs articulaires inflammatoires, ou d'autre douleurs inflammatoires (e.g. hygroma, tendinite,..), les maladies fibrotiques, la fibrose idiopathique pulmonaire, la fibrose cystique, la glumérulonéphrite, la spondylite rhumatoïde, la goutte, la résorption osseuse et cartilagineuse, l'ostéoporose, la maladie de Paget, le myélome multiple, l'uvéorétinite, les chocs septiques, la septicémie, les chocs endotoxiniques, la réaction du greffon contre l'hôte, le rejet des greffes, le syndrome de détresse respiratoire de l'adulte, la silicose, l'asbestose, la sarcoïdose pulmonaire, la maladie de Crohn, la colite ulcérative, la sclérose latérale amyotrophique, la maladie d'Alzheimer, la maladie de Parkinson, le lupus érythémateux disséminé, les chocs hémodynamiques, les pathologies ischémiques (infarctus myocardique, ischémie myocardique, vasospasme coronarien, angine de poitrine, insuffisance cardiaque, attaque cardiaque), les atteintes post ischémiques de reperfusion, la malaria, les infections mycobactériennes, la méningite, la lèpre, les infections virales (HIV, cytomégalovirus, virus de l'herpès), les infections opportunistes liées au Sida, la tuberculose, le psoriasis, la dermatose atopique et de contact, le diabète, la cachexie, le cancer, les dommages liés aux radiations.

Selon un de ses aspects, la présente invention concerne un composé selon l'invention, ou un sel pharmaceutiquement acceptable dudit composé, pour le traitement des maladies ci-dessus mentionnées.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, intraarticulaire, topique, locale, intratrachéale, intranasale, transdermique ou rectale, le principe actif de formule (I) ci-dessus, ou son sel peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intraarticulaire, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Par voie orale, la dose de principe actif administrée par jour peut atteindre 0,01 à 100 mg/kg, en une ou plusieurs prises, préférentiellement 0,02 à 50 mg/kg.

A titre d'exemple, une forme unitaire d'administration d'un composé selon l'invention sous forme de solution ou suspension intraarticulaire peut comprendre les composants suivants :
Composé selon l'invention

| | |
|---|---|
| Polyvinylpyrrolidone (PVP) K17 | 2% |
| Lutrol F68 | 1% |
| NaCl | 0,9% |

Par voie intraarticulaire, la dose de principe actif administrée peut atteindre 0,01 à 40 mg/kg par articulation, préférentiellement la fréquence des injections est séparée d'au moins un mois.

A titre d'exemple additionnel, une forme unitaire d'administration d'un composé selon l'invention sous forme de solution ou suspension intraarticulaire peut comprendre les composants suivants :

| | |
|---|---|
| Composé selon l'invention | 48 mg |
| Polyvinylpyrrolidone (PVP) | 20 mg |
| Polaxamer 188 | 10 mg |
| NaCl | 9 mg |
| 0,1 N NaOH/0,1 N HCl | q.s. pH ajusté à 6,8-7,4 |
| Eau pour injection | 1000 mg |

Par voie intraarticulaire, la dose de principe actif administrée peut atteindre 4ng à 96 mg par articulation, préférentiellement la fréquence des injections est séparée d'au moins un mois.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables.

Selon un autre de ses aspects, l'invention concerne une méthode de traitement des maladies liées à des troubles immunitaires et inflammatoires ainsi que dans le traitement de la douleur, notamment l'athérosclérose, les maladies auto-immunes, les maladies qui entraînent la démyélinisation des neurones (telles que la sclérose en plaques), l'asthme, l'arthrite rhumatoïde et ses douleurs articulaires, l'ostéoarthrite et ses douleurs articulaires ainsi que d'autres douleurs articulaires inflammatoires, ou d'autre douleurs inflammatoires (e.g. hygroma, tendinite,..), les maladies fibrotiques, la fibrose idiopathique pulmonaire, la fibrose cystique, la glumérulonéphrite, la spondylite rhumatoïde, la goutte, la résorption osseuse et cartilagineuse, l'ostéoporose, la maladie de Paget, le myélome multiple, l'uvéorétinite, les chocs septiques, la septicémie, les chocs endotoxiniques, la réaction du greffon contre l'hôte, le rejet des greffes, le syndrome de détresse respiratoire de l'adulte, la silicose, l'asbestose, la sarcoïdose pulmonaire, la maladie de Crohn, la colite ulcérative, la sclérose latérale amyotrophique, la maladie d'Alzheimer, la maladie de Parkinson, le lupus érythémateux disséminé, les chocs hémodynamiques, les pathologies ischémiques (infarctus myocardique, ischémie myocardique, vasospasme coronarien, angine de poitrine, insuffisance cardiaque, l'attaque cardiaque), les atteintes post ischémiques de reperfusion, la malaria, les infections mycobactériennes, la méningite, la lèpre, les infections virales (HIV, cytomegalovirus, virus de l'herpès), les infections opportunistes liées au Sida, la tuberculose, le psoriasis, la dermatose atopique et de contact, le diabète, la cachexie, le cancer, les dommages liés aux radiations, comprenant l'administration d'un composé de formule (I) ou de l'un de ses sels pharmaceutiquement acceptables, seul ou en association avec d'autres principes actifs.

Selon un autre de ses aspects, l'invention concerne une méthode de traitement des douleurs ou inflammations articulaires, plus particulièrement celles de l'épaule, du genou ou des doigts comprenant l'administration d'un composé de formule (I) ou de l'un de ses sels pharmaceutiquement acceptables, seul ou en association avec d'autres principes actifs. L'invention concerne également une méthode de traitement, telle que mentionnée ci-dessus, où l'administration d'un composé de formule (I) ou de l'un de ses sels pharmaceutiquement acceptables, seul ou en association avec d'autres principes actifs se fait par injection intraarticulaire.

Selon un autre de ses aspects, l'invention concerne l'utilisation d'un composé de formule (I) ou un de ses sels pharmaceutiquement acceptables pour la préparation d'un médicament destiné au traitement des douleurs ou inflammations articulaires ou plus particulièrement, l'utilisation d'un composé de formule (I) ou un de ses sels pharmaceutiquement acceptables caractérisée en ce que le médicament est injecté dans l'articulation.

Selon un autre de ses aspects, l'invention concerne un composé de formule (I) ou un de ses sels pharmaceutiquement acceptables pour le traitement des douleurs ou inflammations articulaires ou plus particulièrement, le traitement caractérisée en ce que le médicament est injecté dans l'articulation.

## Revendications

1. Composé de formule (I) : 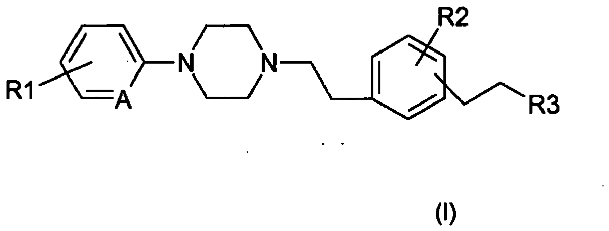 dans laquelle :
- R1 et R2 représentent, indépendamment l'un de l'autre, un atome d'hydrogène, un atome d'halogène, un groupe (C1-C5)alkyle, un groupe (C1-C5)haloalkyle, un groupe (C1-C2)perfluoroalkyle, un groupe (C1-C5)alcoxy ou un groupe (C1-C2)perfluoroalcoxy;
- R3 représente un groupe (C1-C5)alkyle ;
- A représente =CH- ou =N-;
à l'état de base ou de sel d'addition à un acide.

2. Composé selon la revendication 1 tel que
- R1 représente un groupe (C1-C5)haloalkyle ou un groupe (C1-C2)perfluoroalkyle; à l'état de base ou de sel d'addition à un acide.

3. Composé selon la revendication 2 tel que
- R1 représente un groupe (C1-C5)fluoroalkyle; à l'état de base ou de sel d'addition à un acide.

4. Composé selon la revendication 1, 2 ou 3 **caractérisé en ce que** :
- R2 représente un atome d'hydrogène ou un groupe (C1-C5)alkyle; à l'état de base ou de sel d'addition à un acide.

5. Composé selon la revendication 1, 2, 3 ou 4 tel que la pipérazine est liée par le groupe éthyle en position 3 sur le groupe phényle: 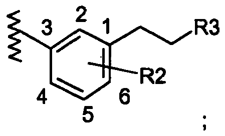 à l'état de base ou de sel d'addition à un acide.

6. Composé selon l'une quelconque des revendications précédentes tel que
- R1 représente un groupe (C1-C2)perfluoroalkyle; à l'état de base ou de sel d'addition à un acide.

7. Composé selon l'une quelconque des revendications précédentes tel que
- R2 représente un atome d'hydrogène ou un groupe (C1-C3)alkyle à l'état de base ou de sel d'addition à un acide.

8. Composé selon l'une quelconque des revendications précédentes **caractérisé en ce qu'**il consiste en le:
1-[2-(4-Méthyl-3-pentyl-phényl)-éthyl]-4-(3-trifluorométhyl-phényl)-pipérazine ; à l'état de base ou de sel d'addition à un acide.

9. Composé selon l'une quelconque des revendications précédentes choisi parmi:
- composé n°1 :l'hémipamoate de 1-(3-trifluorométhyl-phényl)-4-[2-(4-Méthyl-3-pentyl-phényl)-éthyl]-pipérazine ;
- composé n°1 bis:l'hémipamoate de 1-(3-trifluorométhyl-phényl)-4-[2-(4-Méthyl-3-pentyl-phényl)-éthyl]-pipérazine en mélange avec 0, 5 mole d'acide pamoïque libre ;
- composé n°2 : le chlorhydrate de 1-(3-trifluorométhyl-phényl)-4-[2-(4-Méthoxy-3-pentyl-phényl)-éthyl]-pipérazine ;
- composé n°3 : le chlorhydrate de 1-(3-trifluorométhyl-phényl)-4-[2-(4-Méthyl-3-propyl-phényl)-éthyl]-pipérazine ;
- composé n°4 : le chlorhydrate de 1-(3-fluoro-phényl)-4-[2-(4-Méthyl-3-pentyl-phényl)-éthyl]-pipérazine ;
- composé n°5 : le chlorhydrate de 1-(3-trifluorométhyl-phényl)-4-[2-(4-Méthyl-3-heptyl-phényl)-éthyl]-pipérazine ;
- composé n°6 : le chlorhydrate de 1-(6-trifluorométhyl-pirid-2-yl)-4-[2-(4-Méthyl-3-pentyl-phényl)-éthyl]-pipérazine ;
- composé n°7 : le chlorhydrate de 1-(3-fluoro-phényl)-4-[2-(4-Méthoxy-3-pentyl-phényl)-éthyl]-pipérazine ;
- composé n°8 : le chlorhydrate de 1-(3-difluorométhyl-phényl)-4-[2-(4-Méthyl-3-pentyl-phényl)-éthyl]-piperazine ;
- composé n°9 : le chlorhydrate de 1-(phényl)-4-[2-(4-Méthyl-3-pentyl-phényl)-éthyl]-pipérazine ;
- composé n°10: le chlorhydrate de 1-(3-méthoxy-phényl)-4-[2-(4-Méthyl-3-pentyl-phényl)-éthyl]-pipérazine ;
- composé n°11 : le chlorhydrate de 1-(3-trifluorométhyl-phényl)-4-[2-(4-Méthyl-3-(2-méthylbutyl)-phényl)-éthyl]-pipérazine ;
- composé n°12 : le chlorhydrate de 1-(3-difluorométhyl-phényl)-4-[2-(4-Méthoxy-3-pentyl-phényl)-éthyl]-pipérazine ;
- composé n°13.: le chlorhydrate de 1-(3-trifluorométhyl-phényl)-4-[2-(4-Méthoxy-3-heptyl-phényl)-éthyl]-pipérazine ;
- composé n°14 : le chlorhydrate de 1-(3-trifluorométhyl-phényl)-4-[2-(4-Méthyl-3-hexyl-phényl)-éthyl]-pipérazine ;
- composé n°15 : le chlorhydrate de 1-(3-trifluorométhyl-phényl)-4-[2-(4-Méthoxy-3-propyl-phényl)-éthyl]-pipérazine ;
- composé n°16 : le chlorhydrate de 1-(phényl)-4-[2-(4-Méthoxy-3-pentyl-phényl)-éthyl]-pipérazine ;
- composé n°17 : le chlorhydrate de 1- (4-chloro-phényl)-4-[2-(4-Méthyl-3-pentyl-phényl)-éthyl]-pipérazine ;
- composé n°18 : le chlorhydrate de 1-(3-trifluorométhyl-phényl)-4-[2-(4-Méthyl-3-pentyl-phényl)-éthyl]-pipérazine ;
- composé n°19 : le benzène sulphonate de 1-(3-trifluorométhyl-phényl)-4-[2-(4-Méthyl-3-pentyl-phényl)-éthyl]-pipérazine ;
- composé n°20 : le 2-naphtalène sulphonate de 1-(3-trifluorométhyl-phényl)-4-[2-(4-Méthyl-3-pentyl-phényl)-éthyl]-pipérazine ;
- composé n°21 : le p-Tolyl-sulphonate de 1-(3-trifluorométhyl-phényl)-4-[2-(4-Méthyl-3-pentyl-phényl)-éthyl]-pipérazine ;
- composé n°22 :l'hémi-2,5-naphtaléne-disulphonate de 1-(3-trifluorométhyl-phényl)-4-[2-(4-Méthyl-3-pentyl-phényl)-éthyl]-pipérazine ;
- composé n°23 : le dichlorydrate de 1-(4-Fluoro-phényl)-4-[2-(4-méthyl-3-pentyl-phényl)-éthyl]-pipérazine ;
- composé n°24: la base de 1-(4-Méthoxy-phényl)-4-[2-(4-méthyl-3-pentyl-phényl)-éthyl]-pipérazine ;
- composé n°25: le dichlorydrate de 1-(5-Bromo-pyridin-2-yl)-4-[2-(4-méthyl-3-pentyl-phényl)-éthyl]-pipérazine ;
- composé n°26 : le dichlorydrate de 1-[2-(4-Méthyl-3-pentyl-phényl)-éthyl]-4-(5-trifluorométhyl-pyridin-2-yl)-pipérazine;
- composé n°27: le chlorydrate de 1-(4-tert-Butyl-phényl)-4-[2-(4-méthyl-3-pentyl-phényl)-éthyl]-pipérazine ;
- composé n°28: le dichlorydrate de 1-(4-Ethoxy-phényl)-4-[2-(4-méthyl-3-pentyl-phényl)-éthyl]-pipérazine ;
- composé n°29 : le dichlorydrate de 1-[2-(4-Méthyl-3-pentyl-phényl)-éthyl]-4-m-tolyl-pipérazine ;
- composé n°30: le dichlorydrate de 1-[2-(4-Méthyl-3-pentyl-phényl)-éthyl]-4-pyridin-2-yl-pipérazine ;
- composé n°31 : le chlorydrate de 1-(6-Bromo-pyridin-2-yl)-4-[2-(4-méthyl-3-pentyl-phényl)-éthyl]-pipérazine ;
- composé n°32 : le chlorydrate de 1-(2-Chloro-phényl)-4-[2-(4-méthyl-3-pentyl-phényl)-éthyl]-pipérazine ;
- composé n°33 : le chlorydrate de 1-(2-méthyl-phényl)-4-[2-(4-méthyl-3-pentyl-phényl)-éthyl]-pipérazine ;
- composé n°34 : le chlorydrate de 1-(3-trifluorométhoxyl-phényl)-4-[2-(4-méthyl-3-pentyl-phényl)-éthyl]-pipérazine ;
- composé n°35: le chlorydrate de 1-(5-Chloro-pyridin-2-yl)-4-[2-(4-méthyl-3-pentyl-phényl)-éthyl]-pipérazine ;
- composé n°36: l'oxalate de 1-(3-trifluorométhyl-phényl)-4-[2-(4-Méthyl-3-pentyl-phényl)-éthyl]-pipérazine ;
- composé n°37 : le fumarate de 1-(3-trifluorométhyl-phényl)-4-[2-(4-Méthyl-3-pentyl-phényl)-éthyl]-pipérazine ;
- composé n°38 : le succinate de 1-(3-trifluorométhyl-phényl)-4-[2-(4-Méthyl-3-pentyl-phényl)-éthyl]-pipérazine ;
- composé n°39 : le dihippurate de 1-(3-trifluorométhyl-phényl)-4-[2-(4-Méthyl-3-pentyl-phényl)-éthyl]-pipérazine.

10. Procédé de préparation d'un composé selon l'une quelconque des revendications précédentes comprenant l'étape consistant en la condensation d'un composé de formule (II). 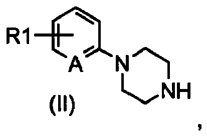 dans laquelle R1 et A sont tels que définis dans la revendication 1, avec un composé de formule (III) : 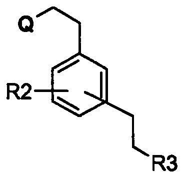 dans laquelle R2 et R3 sont tels que définis dans la revendication 1 et Q représente un groupe partant et éventuellement en sa transformation en un de ses sels.

11. Composition pharmaceutique contenant en tant que principe actif un composé de formule (I) selon l'une quelconque des revendications de 1 à 9 ou un de ses sels pharmaceutiquement acceptables.

12. Médicament comprenant en tant que principe actif un composé de formule (I) selon les revendications de 1 à 9 ou un de ses sels pharmaceutiquement acceptables.

13. Utilisation d'un composé de formule (I) selon l'une quelconque des revendications de 1 à 9 ou un de ses sels pharmaceutiquement acceptables pour la préparation d'un médicament destiné au traitement ou la prévention de la douleur et/ou des maladies liées à des troubles immunitaires et inflammatoires.

14. Utilisation d'un composé de formule (I) ou un de ses sels pharmaceutiquement acceptables selon la revendication 13 pour la préparation d'un médicament destiné au traitement des douleurs ou inflammations articulaires.

15. Utilisation d'un composé de formule (I) un de ses sels pharmaceutiquement acceptables selon la revendication 14 **caractérisé en ce que** le médicament est injecté dans l'articulation.

## Patentansprüche

1. Verbindung der Formel (I): 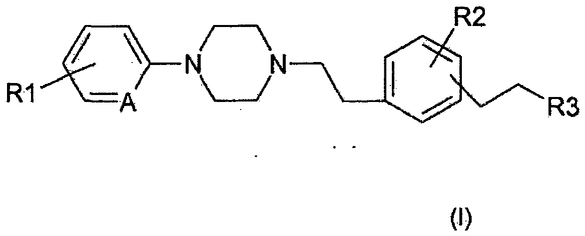 worin:
- R1 und R2 unabhängig voneinander für ein Wasserstoffatom, ein Halogenatom, eine (C1-C5)-Alkylgruppe, eine (C1-C5)-Halogenalkylgruppe, eine (C1-C2)-Perfluoralkylgruppe, eine (C1-C5)-Alkoxygruppe oder eine (C1-C2)-Perfluoralkoxygruppe stehen;
- R3 für eine (C1-C5)-Alkylgruppe steht;
- A für =CH- oder =N- steht;
in Basen- oder Säureadditionssalzform.

2. Verbindung nach Anspruch 1, wobei
- R1 für eine (C1-C5)-Halogenalkylgruppe oder eine (C1-C2)-Perfluoralkylgruppe steht; in Basen- oder Säureadditionssalzform.

3. Verbindung nach Anspruch 2, wobei
- R1 für eine (C1-C5)-Fluoralkylgruppe steht; in Basen- oder Säureadditionssalzform.

4. Verbindung nach Anspruch 1, 2 oder 3, **dadurch gekennzeichnet, dass**:
- R2 für ein Wasserstoffatom oder eine (C1-C5)-Alkylgruppe steht; in Basen- oder Säureadditionssalzform.

5. Verbindung nach Anspruch 1, 2, 3 oder 4, wobei das Piperazin über die Ethylgruppe in Position 3 an die Phenylgruppe gebunden ist: 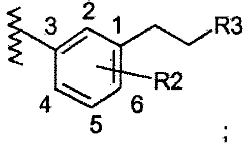 in Basen- oder Säureadditionssalzform.

6. Verbindung nach einem der vorhergehenden Ansprüche, wobei
- R1 für eine (C1-C2)-Perfluoralkylgruppe steht; in Basen- oder Säureadditionssalzform.

7. Verbindung nach einem der vorhergehenden Ansprüche, wobei
- R2 für ein Wasserstoffatom oder eine (C1-C3)-Alkylgruppe steht; in Basen- oder Säureadditionssalzform.

8. Verbindung nach einem der vorhergehenden Ansprüche, **dadurch gekennzeichnet, dass** es sich um:
1-[2-(4-Methyl-3-pentylphenyl)ethyl]-4-(3-trifluormethylphenyl)piperazin handelt;
in Basen- oder Säureadditionssalzform.

9. Verbindung nach einem der vorhergehenden Ansprüche, ausgewählt aus:
- Verbindung Nr. 1: 1-(3-Trifluormethylphenyl)-4-[2-(4-methyl-3-pentylphenyl)ethyl]piperazin- hemipamoat;
- Verbindung Nr. 1 bis: 1-(3-Trifluormethylphenyl)-4-[2-(4-methyl-3-pentylphenyl)ethyl]-piperazin-hemipamoat als Mischung mit 0,5 mol freier Pamoasäure;
- Verbindung Nr. 2: 1-(3-Trifluormethylphenyl)-4-[2-(4-methoxy-3-pentylphenyl)ethyl]piperazin-hydrochlorid;
- Verbindung Nr. 3: 1-(3-Trifluormethylphenyl)-4-[2-(4-methyl-3-propylphenyl)ethyl]piperazin-hydrochlorid;
- Verbindung Nr. 4: 1-(3-Fluorphenyl)-4-[2-(4-methyl-3-pentylphenyl)ethyl]piperazin-hydrochlorid;
- Verbindung Nr. 5: 1-(3-Trifluormethylphenyl)-4-[2-(4-methyl-3-heptylphenyl)ethyl]piperazin-hydrochlorid;
- Verbindung Nr. 6: 1-(6-Trifluormethylpyrid-2-yl)-4-[2-(4-methyl-3-pentylphenyl)ethyl]piperazin-hydrochlorid;
- Verbindung Nr. 7: 1-(3-Fluorphenyl)-4-[2-(4-methoxy-3-pentylphenyl)ethyl]piperazin-hydrochlorid;
- Verbindung Nr. 8: 1-(3-Difluormethylphenyl)-4-[2-(4-methyl-3-pentylphenyl)ethyl]piperazin-hydrochlorid;
- Verbindung Nr. 9: 1-(Phenyl)-4-[2-(4-methyl-3-pentylphenyl)ethyl]piperazin-hydrochlorid;
- Verbindung Nr. 10: 1-(3-Methoxyphenyl)-4-[2-(4-methyl-3-pentylphenyl)ethyl]piperazin-hydrochlorid;
- Verbindung Nr. 11: 1-(3-Trifluormethylphenyl)-4-[2-(4-methyl-3-(2-methylbutyl)phenyl)ethyl]-piperazin-hydrochlorid;
- Verbindung Nr. 12: 1-(3-Difluormethylphenyl)-4-[2-(4-methoxy-3-pentylphenyl)ethyl]piperazin-hydrochlorid;
- Verbindung Nr. 13.: 1-(3-Trifluormethylphenyl)-4-[2-(4-methoxy-3-heptylphenyl)ethyl]piperazin-hydrochlorid;
- Verbindung Nr. 14: 1-(3-Trifluormethylphenyl)-4-[2-(4-methyl-3-hexylphenyl)ethyl]piperazin-hydrochlorid;
- Verbindung Nr. 15: 1-(3-Trifluormethylphenyl)-4-[2-(4-methoxy-3-propylphenyl)ethyl]piperazin-hydrochlorid;
- Verbindung Nr. 16: 1-(Phenyl)-4-[2-(4-methoxy-3-pentylphenyl)ethyl]piperazin-hydrochlorid;
- Verbindung Nr. 17: 1-(4-Chlorphenyl)-4-[2-(4-methyl-3-pentylphenyl)ethyl]piperazin-hydrochlorid;
- Verbindung Nr. 18: 1-(3-Trifluormethylphenyl)-4-[2-(4-methyl-3-pentylphenyl)ethyl]piperazin-hydrochlorid;
- Verbindung Nr. 19: 1-(3-Trifluormethylphenyl)-4-[2-(4-methyl-3-pentylphenyl)ethyl]piperazin-benzolsulfonat;
- Verbindung Nr. 20: 1-(3-Trifluormethylphenyl)-4-[2-(4-methyl-3-pentylphenyl)ethyl]piperazin-2-naphthalinsulfonat;
- Verbindung Nr. 21: 1-(3-Trifluormethylphenyl)-4-[2-(4-methyl-3-pentylphenyl)ethyl]piperazin-p-tolylsulfonat;
- Verbindung Nr. 22: 1-(3-Trifluormethylphenyl)-4-[2-(4-methyl-3-pentylphenyl)ethyl]piperazin-hemi-2,5-naphthalindisulfonat;
- Verbindung Nr. 23: 1-(4-Fluorphenyl)-4-[2-(4-methyl-3-pentylphenyl)ethyl]piperazin-dihydrochlorid;
- Verbindung Nr. 24: 1-(4-Methoxyphenyl)-4-[2-(4-methyl-3-pentylphenyl)ethyl]piperazin (Base);
- Verbindung Nr. 25: 1-(5-Brompyridin-2-yl)-4-[2-(4-methyl-3-pentylphenyl)ethyl]piperazin-dihydrochlorid;
- Verbindung Nr. 26: 1-[2-(4-Methyl-3-pentylphenyl)ethyl]-4-(5-trifluormethylpyridin-2-yl)-piperazin-dihydrochlorid;
- Verbindung Nr. 27: 1-(4-tert.-Butylphenyl)-4-[2-(4-methyl-3-pentylphenyl)ethyl]piperazin-hydrochlorid;
- Verbindung Nr. 28: 1-(4-Ethoxyphenyl)-4-[2-(4-methyl-3-pentylphenyl)ethyl]piperazin-dihydrochlorid;
- Verbindung Nr. 29: 1-[2-(4-Methyl-3-pentylphenyl)ethyl]-4-m-tolylpiperazin-dihydrochlorid;
- Verbindung Nr. 30: 1-[2-(4-Methyl-3-pentylphenyl)ethyl]-4-pyridin-2-ylpiperazin-dihydrochlorid;
- Verbindung Nr. 31: 1-(6-Brompyridin-2-yl)-4-[2-(4-methyl-3-pentylphenyl)ethyl]piperazin-hydrochlorid;
- Verbindung Nr. 32: 1-(2-Chlorphenyl)-4-[2-(4-methyl-3-pentylphenyl)ethyl]piperazin-hydrochlorid;
- Verbindung Nr. 33: 1-(2-Methylphenyl)-4-[2-(4-methyl-3-pentylphenyl)ethyl]piperazin-hydrochlorid;
- Verbindung Nr. 34: 1-(3-Trifluormethoxylphenyl)-4-[2-(4-methyl-3-pentylphenyl)ethyl]piperazin-hydrochlorid;
- Verbindung Nr. 35: 1-(5-Chlorpyridin-2-yl)-4-[2-(4-methyl-3-pentylphenyl)ethyl]piperazin-hydrochlorid;
- Verbindung Nr. 36: 1-(3-Trifluormethylphenyl)-4-[2-(4-methyl-3-pentylphenyl)ethyl]piperazin-oxalat;
- Verbindung Nr. 37: 1-(3-Trifluormethylphenyl)-4-[2-(4-methyl-3-pentylphenyl)ethyl]piperazin-fumarat;
- Verbindung Nr. 38: 1-(3-Trifluormethylphenyl)-4-[2-(4-methyl-3-pentylphenyl)ethyl]piperazin-succinat;
- Verbindung Nr. 39: 1-(3-Trifluormethylphenyl)-4-[2-(4-methyl-3-pentylphenyl)ethyl]piperazin-dihippurat.

10. Verfahren zur Herstellung einer Verbindung nach einem der vorhergehenden Ansprüche, bei dem man eine Verbindung der Formel (II) 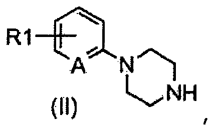 worin R1 und A wie in Anspruch 1 definiert sind, mit einer Verbindung der Formel (III): 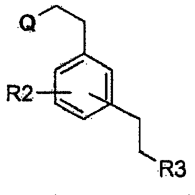 worin R2 und R3 wie in Anspruch 1 definiert sind und Q für eine Abgangsgruppe steht, umsetzt und gegebenenfalls in ein Salz davon umwandelt.

11. Pharmazeutische Zusammensetzung, enthaltend als Wirkstoff eine Verbindung der Formel (I) nach einem der Ansprüche 1 bis 9 oder ein pharmazeutisch unbedenkliches Salz davon.

12. Medikament, umfassend als Wirkstoff eine Verbindung der Formel (I) nach einem der Ansprüche 1 bis 9 oder ein pharmazeutisch unbedenkliches Salz davon.

13. Verwendung einer Verbindung der Formel (I) nach einem der Ansprüche 1 bis 9 oder eines pharmazeutisch unbedenklichen Salzes davon zur Herstellung eines Medikaments zur Behandlung oder Prävention von Schmerzen und/oder mit Immun- und Entzündungsstörungen verbundenen Erkrankungen.

14. Verwendung einer Verbindung der Formel (I) oder eines pharmazeutisch unbedenklichen Salzes davon nach Anspruch 13 zur Herstellung eines Medikaments zur Behandlung von Gelenkschmerzen oder -entzündungen.

15. Verwendung einer Verbindung der Formel (I) oder eines pharmazeutisch unbedenklichen Salzes davon nach Anspruch 14, **dadurch gekennzeichnet, dass** das Medikament in das Gelenk injiziert wird.

## Claims

1. Compound of formula (I): 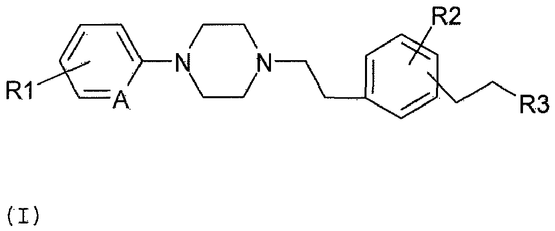 in which:
- R1 and R2 represent, independently of each other, a hydrogen atom, a halogen atom, a (C1-C5)alkyl group, a (C1-C5)haloalkyl group, a (C1-C2)perfluoroalkyl group, a (C1-C5)alkoxy group or a (C1-C2)perfluoroalkoxy group;
- R3 represents a (C1-C5)alkyl group;
- A represents =CH- or =N-;
in the form of the base or an acid-addition salt.

2. Compound according to Claim 1, wherein
- R1 represents a (C1-C5)haloalkyl group or a (C1-C2)perfluoroalkyl group; in the form of the base or an acid-addition salt.

3. Compound according to Claim 2, wherein
- R1 represents a (C1-C5)fluoroalkyl group; in the form of the base or an acid-addition salt.

4. Compound according to Claim 1, 2 or 3, **characterized in that**:
- R2 represents a hydrogen atom or a (C1-C5)alkyl group; in the form of the base or an acid-addition salt.

5. Compound according to Claim 1, 2, 3 or 4, wherein the piperazine is linked via the ethyl group in position 3 on the phenyl group: 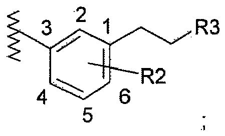 in the form of the base or an acid-addition salt.

6. Compound according to any one of the preceding Claims, wherein
- R1 represents a (C1-C2)perfluoroalkyl group; in the form of the base or an acid-addition salt.

7. Compound according to any one of the preceding Claims, wherein
- R2 represents a hydrogen atom or a (C1-C3)alkyl group in the form of the base or an acid-addition salt.

8. Compound according to any one of the preceding Claims, **characterized in that** it is:
1-[2-(4-methyl-3-pentylphenyl)ethyl]-4-(3-trifluoromethylphenyl)piperazine;
in the form of the base or an acid-addition salt.

9. Compound according to any one of the preceding Claims, chosen from:
- compound 1: 1-(3-trifluoromethylphenyl)-4-[2-(4-methyl-3-pentylphenyl)ethyl]piperazine hemipamoate;
- compound 1 bis: 1-(3-trifluoromethylphenyl)-4-[2-(4-methyl-3-pentylphenyl) ethyl] piperazine hemipamoate as a mixture with 0.5 mol of free pamoic acid;
- compound 2: 1-(3-trifluoromethylphenyl)-4-[2-(4-methoxy-3-pentylphenyl)ethyl]piperazine hydrochloride;
- compound 3: 1-(3-trifluoromethylphenyl)-4-[2-(4-methyl-3-propylphenyl)ethyl]piperazine hydrochloride;
- compound 4: 1-(3-fluorophenyl)-4-[2-(4-methyl-3-pentylphenyl)ethyl]piperazine hydrochloride;
- compound 5: 1-(3-trifluoromethylphenyl)-4-[2-(4-methyl-3-heptylphenyl)ethyl]piperazine hydrochloride;
- compound 6: 1-(6-trifluoromethylpyrid-2-yl)-4-[2-(4-methyl-3-pentylphenyl)ethyl]piperazine hydrochloride ;
- compound 7: 1-(3-fluorophenyl)-4-[2-(4-methoxy-3-pentylphenyl)ethyl]piperazine hydrochloride;
- compound 8: 1-(3-difluoromethylphenyl)-4-[2-(4-methyl-3-pentylphenyl)ethyl]piperazine hydrochloride;
- compound 9: 1-(phenyl)-4-[2-(4-methyl-3-pentylphenyl)ethyl]piperazine hydrochloride;
- compound 10: 1-(3-methoxyphenyl)-4-[2-(4-methyl-3-pentylphenyl)ethyl]piperazine hydrochloride;
- compound 11: 1-(3-trifluoromethylphenyl)-4-[2-(4-methyl-3-(2-methylbutyl)phenyl)ethyl]piperazine hydrochloride;
- compound 12: 1-(3-difluoromethylphenyl)-4-[2-(4-methoxy-3-pentylphenyl)ethyl]piperazine hydrochloride;
- compound 13.: 1-(3-trifluoromethylphenyl)-4-[2-(4-methoxy-3-heptylphenyl)ethyl]piperazine hydrochloride;
- compound 14: 1-(3-trifluoromethylphenyl)-4-[2-(4-methyl-3-hexylphenyl)ethyl]piperazine hydrochloride;
- compound 15: 1-(3-trifluoromethylphenyl)-4-[2-(4-methoxy-3-propylphenyl)ethyl]piperazine hydrochloride;
- compound 16: 1-(phenyl)-4-[2-(4-methoxy-3-pentylphenyl)ethyl]piperazine hydrochloride;
- compound 17: 1-(4-chlorophenyl)-4-[2-(4-methyl-3-pentylphenyl)ethyl]piperazine hydrochloride;
- compound 18 : 1-(3-trifluoromethylphenyl)-4-[2-(4-methyl-3-pentylphenyl)ethyl]piperazine hydrochloride;
- compound 19: 1-(3-trifluoromethylphenyl)-4-[2-(4-methyl-3-pentylphenyl)ethyl]piperazine benzenesulfonate;
- compound 20: 1-(3-trifluoromethylphenyl)-4-[2-(4-methyl-3-pentylphenyl)ethyl]piperazine 2-naphthalenesulfonate;
- compound 21: 1-(3-trifluoromethylphenyl)-4-[2-(4-methyl-3-pentylphenyl)ethyl]piperazine p-tolylsulfonate;
- compound 22: 1-(3-trifluoromethylphenyl)-4-[2-(4-methyl-3-pentylphenyl)ethyl]piperazine hemi-2,5-naphthalenedisulfonate;
- compound 23: 1-(4-fluorophenyl)-4-[2-(4-methyl-3-pentylphenyl)ethyl]piperazine dihydrochloride;
- compound 24: 1-(4-methoxyphenyl)-4-[2-(4-methyl-3-pentylphenyl)ethyl]piperazine (base);
- compound 25: 1-(5-bromopyrid-2-yl)-4-[2-(4-methyl-3-pentylphenyl)ethyl]piperazine dihydrochloride;
- compound 26: 1-[2-(4-methyl-3-pentylphenyl)ethyl]-4-(5-trifluoromethylpyrid-2-yl)piperazine dihydrochloride;
- compound 27: 1-(4-tert-butylphenyl)-4-[2-(4-methyl-3-pentylphenyl)ethyl]piperazine hydrochloride;
- compound 28: 1-(4-ethoxyphenyl)-4-[2-(4-methyl-3-pentylphenyl)ethyl]piperazine dihydrochloride;
- compound 29: 1-[2-(4-methyl-3-pentylphenyl)ethyl]-4-m-tolylpiperazine dihydrochloride;
- compound 30: 1-[2-(4-methyl-3-pentylphenyl)ethyl]-4-pyrid-2-ylpiperazine dihydrochloride;
- compound 31: 1-(6-bromopyrid-2-yl)-4-[2-(4-methyl-3-pentylphenyl)ethyl]piperazine hydrochloride;
- compound 32: 1- (2-chlorophenyl) -4- [2-(4-methyl-3-pentylphenyl)ethyl]piperazine hydrochloride;
- compound 33: 1-(2-methylphenyl)-4-[2-(4-methyl-3-pentylphenyl)ethyl]piperazine hydrochloride; compound 34: 1-(3-trifluoromethoxylphenyl)-4-[2-(4-methyl-3-pentylphenyl)ethyl]piperazine hydrochloride;
- compound 35: 1-(5-chloropyrid-2-yl)-4-[2-(4-methyl-3-pentylphenyl)ethyl]piperazine hydrochloride;
- compound 36: 1-(3-trifluoromethylphenyl)-4-[2-(4-methyl-3-pentylphenyl)ethyl]piperazine oxalate;
- compound 37: 1-(3-trifluoromethylphenyl)-4-[2-(4-methyl-3-pentylphenyl)ethyl]piperazine fumarate;
- compound 38: 1-(3-trifluoromethylphenyl)-4-[2-(4-methyl-3-pentylphenyl)ethyl]piperazine succinate;
- compound 39: 1-(3-trifluoromethylphenyl)-4-[2-(4-methyl-3-pentylphenyl)ethyl]piperazine dihippurate.

10. Process for preparing a compound according to any one of the preceding claims, comprising the step of condensing a compound of formula (II). 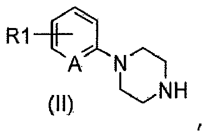 in which R1 and A are as defined according to Claim 1, with a compound of formula (III): 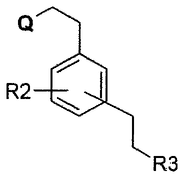 in which R2 and R3 are as defined according to Claim 1 and Q represents a leaving group, and optionally converting it into a salt thereof.

11. Pharmaceutical composition containing, as active principle, a compound of formula (I) according to any one of Claims 1 to 9, or a pharmaceutically acceptable salt thereof.

12. Medicament comprising, as active principle, a compound of formula (I) according to Claims 1 to 9, or a pharmaceutically acceptable salt thereof.

13. Use of a compound of formula (I) according to any one of Claims 1 to 9, or of a pharmaceutically acceptable salt thereof, for the preparation of a medicament for treating or preventing pain and/or diseases associated with immune and inflammatory disorders.

14. Use of a compound of formula (I) or of a pharmaceutically acceptable salt thereof according to Claim 13, for the preparation of a medicament for treating pain or inflammations of the joints.

15. Use of a compound of formula (I) or of a pharmaceutically acceptable salt thereof according to Claim 14, **characterized in that** the medicament is injected into the joints.
